# EUROPEAN PATENT SPECIFICATION

(11) **EP 1 865 814 B1**
(45) Date of publication and mention of the grant of the patent: **19.11.2014**
(21) Application number: 06758192.6
(22) Date of filing: 24.03.2006
(51) Int. Cl.: A47F 7/00, A47C 27/08, A61F 7/02

(54) **THERMOREGULATION DEVICE**
WÄRMEREGULIERUNGSVORRICHTUNG
DISPOSITIF DE THERMOREGULATION

(30) Priority: 25.03.2005 US 665241 P; 25.03.2005 US 665141 P
(43) Date of publication of application: 19.12.2007
(73) Proprietor: Hill-Rom Services, Inc., Batesville IN 47006 (US)
(72) Inventor: KING, Rachel, H., Lawrenceburg, Indiana 47025 (US); YERMANENI, Mayur, Shrewsbury, MA 01545 (US); BRANSON, Gregory, W., Batesville, Indiana 47006 (US); O'NEAL, Todd, Fairfield, Ohio 45014 (US)
(74) Representative: Findlay, Alice Rosemary
(86) International application number: PCT/US2006/010805
(87) International publication number: WO 2006/102598

(56) References cited:
- WO-A-02/29348
- US-A- 1 121 277
- US-A- 2 250 325
- US-A- 2 932 491
- US-A- 3 295 594
- US-A- 3 295 594
- US-A- 3 714 947
- US-A- 3 714 947
- US-A- 4 114 620
- US-A- 4 523 594
- US-A- 4 523 594
- US-A- 4 749 541
- US-A- 5 320 164
- US-A1- 2003 079 488
- US-B1- 6 565 699
- US-B1- 6 942 015

## Description

### BACKGROUND

It is sometimes desirable in the treatment of a patient to either cool or warm the patient. For example, it may be clinically desirable to cool patients who have suffered a stroke or cardiac arrest. In other circumstances, such as when a patient is suffering from hypothermia, it can be desirable to warm the patient.

US 2003/0079488 and US 4114620 disclose patient treatment devices for laying on a patient and having multiple fluid channels. US 1121277 discloses a warming appliance for a bed with channels through the mattress. US 5320164, US 6565699, US 4523594, WO02/29348 and US 3295594 discloses wraps or full garments for heating and/or cooling a wearer, and US 3714947 discloses a baby bunting with panels of stretch fabric and multiple tubes attached to the inner surface of the panels.

### SUMMARY OF THE INVENTION

The invention is defined by the claims.

The invention provides a thermoregulating apparatus for exchanging thermal energy with a patient. The apparatus includes a flexible heat exchange layer and a heat exchange fluid circuit located within the heat exchange layer. The heat exchange layer has a length, a width and a thickness wherein the length and width are both substantially greater than the thickness. The heat exchange layer also defines at least one support surface. The heat exchange fluid circuit includes at least a first segment and a second segment. At least one pressure relief segment is located between the first and second segments of the heat exchange fluid circuit. Differential elongation of the pressure relief section relatively repositions the first and second heat exchange segments in at least one direction when a force is applied to the heat exchange layer in a direction substantially transverse to the support surface of the layer and at a location proximate the pressure relief section and the first and second fluid circuit segments.

A resiliently stretchable material, comprising spandex fibers, is used to form the pressure relief section.

In some embodiments, the first and second heat exchange segments each comprise an elongate conduit. When the heat exchange layer is positioned in a substantially planar orientation at least a portion of one of the conduits defining the first and second heat exchange segments has an arcuate configuration. Differential elongation of the pressure relief section and the first and second heat exchange segments repositions at least a portion of the arcuate configuration into a more linear configuration.

In some embodiments, the heat exchange fluid circuit comprises at least one elongate conduit arranged in a grid including a plurality of nodes. Each of the nodes defines an intersection of a first segment of the conduit with a second segment of the conduit. The first segment is fixed relative to the second segments at each node. When the heat exchange layer is positioned in a substantially planar orientation the nodes are separated by a first set of distances. The first and second segments of the conduit extend between the nodes define a second respective set of lengths, the second set of lengths are greater than the first set of distances allowing the nodes to be repositionable to define a third set of distances separating the nodes that is greater than the first set of distances.

In some embodiments, the fluid structure comprises at least one elongate conduit. The conduit has at least one substantially serpentine portion. When the heat exchange layer is positioned in a substantially planar orientation, the serpentine portion defines a plurality of substantially parallel spaced apart linear segments. A plurality of the pressure relief sections are disposed between the linear segments of the conduit.

In some embodiments, the first and second heat exchange segments each comprise an elongate conduit. The thermoregulating apparatus further comprises at least one resiliently stretchable material layer. The at least one stretchable material layer defines a first sleeve and a second sleeve. The first and second heat exchange segments are respectively disposed within the first and second sleeves.

In some embodiments, the thermoregulating apparatus further comprises a support structure underlying the flexible heat exchange layer. The support structure includes a plurality of gas filled support cells. In a further embodiment, the plurality of gas filled support cells comprise a plurality of substantially cylindrical cells. Each of the cells define an axis oriented substantially transverse to the support surface.

In some embodiments, the flexible heat layer comprises first and second layers of the stretchable material. The heat exchange fluid circuit is disposed between the first and second layers of stretchable material.

In some embodiments, the stretchable material is resiliently stretchable to at least about 200% of its original length.

In some embodiments, the thermoregulating apparatus further comprises a support structure underlying the flexible heat exchange layer. The support structure includes a plurality of gas filled support cells. In a further embodiment, the plurality of gas filled support cells comprise a plurality of substantially cylindrical cells. Each of the cells define an axis oriented substantially transverse to the support surface.

In yet another embodiment, a thermoregulating apparatus for exchanging thermal energy with a patient comprises a flexible heat exchange layer, a first sleeve portion, a second sleeve portion, a heat exchange fluid circuit, and a resiliently stretchable material. The flexible heat exchange layer defines a length, a width and a thickness. Each of the length and the width are substantially greater than the thickness and the layer defines at least one support surface. The first sleeve portion and the second sleeve portion are formed within the flexible heat exchange layer. The heat exchange fluid circuit is disposed within the heat exchange layer. The heat exchange fluid circuit includes a first segment and a second segment. The first segment is disposed within the first sleeve portion and the second segment is disposed within the second sleeve portion. The resiliently stretchable material is disposed between the first segment and the second segment

In some embodiments, the flexible heat exchange layer comprises first and second layers of stretchable material. The first and second layers are secured together to define the first and second sleeve portions. At least one of the first and second layers define the resiliently stretchable material disposed between the first segment and the second segment

In some embodiments, the first sleeve portion is disposed substantially parallel to the second sleeve portion. In a further embodiment, the first and second sleeve portions are substantially linear when the flexible heat exchange layer is disposed in a planar configuration.

In some embodiments, the stretchable material is resiliently stretchable to at least about 200% of its original length.

In some embodiments, the thermoregulating apparatus further comprises a support structure underlying the flexible heat exchange layer. The support structure includes a plurality of gas filled support cells. In a further embodiment, the plurality of gas filled support cells comprise a plurality of substantially cylindrical cells. Each of the cells define an axis oriented substantially transverse to the support surface.

Still other embodiments of the invention take the form of a thermoregulating apparatus for exchanging thermal energy with a patient that includes a flexible heat exchange layer and a heat exchange fluid circuit that has at least one elongate conduit arranged in a grid. The grid includes a plurality of nodes with each of the nodes defining an intersection of a first segment of the conduit with a second segment of the conduit with the first segment being fixed relative to the second segment at each node. When the heat exchange layer is positioned in a substantially planar orientation, the nodes are separated by a first set of distances and the first and second segments of the conduit extending between the nodes define a second respective set of lengths. The second set of lengths are greater than the first set of distances to thereby allow the nodes to be repositionable to define a third set of distances separating the nodes that is greater than the first set of distances.

In a further embodiment, the stretchable material is resiliently stretchable to at least about 200% of its original length.

In some embodiments, the first and second segments of the conduit have an arcuate configuration when the flexible heat exchange layer is in a generally planar configuration. In a further embodiment, the conduit has a portion defining a generally sinusoidal configuration.

In some embodiments, the thermoregulating apparatus further comprises a support structure underlying the flexible heat exchange layer. The support structure includes a plurality of gas filled support cells. In a further embodiment, the plurality of gas filled support cells comprise a plurality of substantially cylindrical cells. Each of the cells define an axis oriented substantially transverse to the support surface.

A method of exchanging thermal energy with an object, which is not part of the invention, includes providing a flexible heat exchange layer having a heat exchange fluid circuit disposed therein and including at least one pressure relief section formed by a resiliently stretchable material and positioning an object on the heat exchange layer. The method also includes differentially resiliently elongating the flexible heat exchange layer to support the object thereon in a manner that facilitates equalization of a support pressure generated by the weight of the object and wherein a majority of the resilient elongation of the flexible heat exchange layer is defined by the pressure relief section. A heat exchange medium is circulated through the fluid circuit and thermal energy is exchanged between the object and the heat exchange medium.

In some aspects of the method, the object is a human.

In a further aspect of the method, the stretchable material is resiliently stretchable to at least about 200% of its original length.

In some aspects of the method, the fluid circuit includes an elongate conduit When the heat exchange layer is in a substantially planar configuration, at least a portion of the conduit has an arcuate configuration. The step of resiliently elongating the heat exchange layer includes repositioning the portion of the conduit having an arcuate configuration into a more linear configuration.

In some aspects of the method, the fluid circuit includes at least one conduit arranged in a grid and having a plurality of nodes. The relative position of the nodes redefined by the step of resiliently deforming the heat exchange layer.

In some aspects of the method, the fluid circuit includes at least one elongate conduit having at least one serpentine portion.

In some aspects of the method, the method further comprises the step of providing a support structure to support the heat exchange layer and the object located thereon. The support layer includes a plurality of gas filled support cells. In a further aspect of the method, the plurality of gas filled support cells comprise a plurality of substantially cylindrical cells.

### BRIEF DESCRIPTION OF THE DRAWINGS

The detailed description particularly refers to the accompanying Figs. in which:
Fig. 1 is an exploded perspective view of a hospital bed, a thermoregulating device and a patient.
Fig. 2 is an exploded perspective view of a thermoregulating device.
Fig. 3 is a partially cut away perspective view of a thermoregulating device that has been mounted on a plurality of gas-filled support cells.
Fig. 4 is a perspective view of a fluid circuit for a heat exchange medium mountable in a thermoregulating device.
Fig. 5 is a detail view of a portion of the fluid circuit of Fig. 4.
Fig. 6 is a schematic top view of a portion of the fluid circuit of Fig. 5 showing its deformation under load.
Fig. 7 is a schematic side view of the fluid circuit portion shown in Fig. 6. Fig. 8 is an exploded perspective view of the upper and lower stretchable layers of a thermoregulating device.
Fig. 9 is a partially exploded perspective view of a thermoregulating device having sleeves for receiving fluid circuit conduits.
Fig. 10 is a perspective view of an assembled thermoregulating device similar to that shown in Fig. 9.
Fig. 11 is a schematic illustration of a fluid circuit that can be used with the thermoregulating device shown in Fig. 10.
Fig. 12 is a schematic illustration of an alternative fluid circuit that can be used with the thermoregulating device shown in Fig. 10.
Fig. 13 is an exploded view of another aspect of a thermoregulating device.
Fig. 14 is a schematic depiction of the thermoregulating device of Fig. 13 deforming under load.
Fig. 15 is a schematic side view of the thermoregulating device depicted in Fig. 13.
Fig. 16 is a perspective view of a portion of a heat exchange medium fluid circuit configured in a grid.
Fig. 17 is a top view of a thermoregulating device including the fluid circuit grid of Fig. 16.
Fig. 18 schematically shows a portion of the fluid circuit grid of Fig. 16 in an unstressed configuration.
Fig. 19 schematically depicts the fluid circuit portion of Fig. 18 deformed under load.

Corresponding reference characters indicate corresponding parts throughout the several views. Although the exemplification set out herein illustrates embodiments of the invention, in several forms, the embodiments disclosed below are not intended to be exhaustive or to be construed as limiting the scope of the invention to the precise forms disclosed.

### DETAILED DESCRIPTION OF THE INVENTION

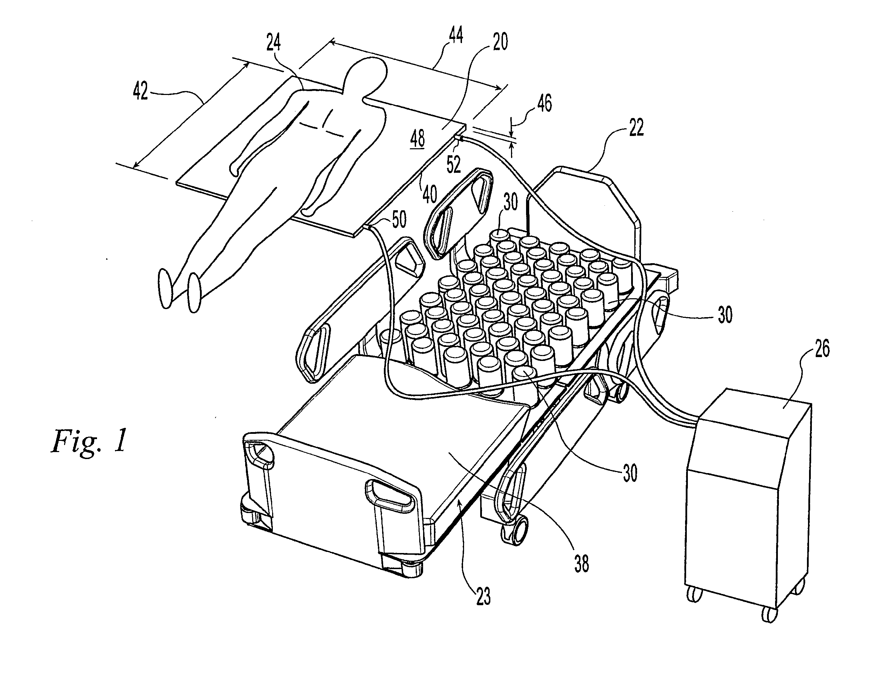

A thermoregulating device 20 for use with a hospital bed 22 having a patient support structure 23 is shown in Fig. 1. A patient 24 may be supported by hospital bed 22. In the treatment of patient 24 it may be desirable to either chill or warm patient 24 and device 20 can be employed for this purpose. A conventional heat exchange unit 26 is coupled with device 20 to circulate a heat exchange medium, e.g., water, through device 20. Heat exchange units 26 are well known in the art and may include a compressor, a condenser and evaporator. Water, or other heat exchange medium employed with device 20, may be chilled by exchanging thermal energy with the evaporator or the water may be warmed by exchanging thermal energy with the condenser. A pump located in unit 26 then circulates the water through device 20.

When a patient is supported on a mattress or similar support structure it is desirable to equalize the support pressure at key pressure points on the patient (e.g., the back of the head or heals of the patient). If the pressure is not spread over a larger area of the patient at these key pressure points, the resulting pressure concentrations increase the likelihood that the patient will experience pressure ulcers or similar undesirable effects. It is known to provide patients with a patient support structure containing gas-filled chambers that facilitate the equalization of the pressure supporting the patient. Such patient support structures can be broadly categorized as powered air surfaces, non-powered air surfaces, or low air loss systems. In general, non-powered air surfaces contain air-filled chambers that do not allow the intentional loss of air from within the chambers and do not require a continuous source of air to be supplied to the chambers. The PrimeAire® ARS Pressure Relief Mattress commercially available from Hill-Rom Company, Inc. having a place of business in Batesville, Indiana is an example of such a non-powered air surface that can be used as support structure 23. Low-air loss systems are a type of powered surface that employ air-filled chambers that are designed to allow a small quantity of air to continuously escape from the chambers during use. An air supply unit is used with such low-air loss systems to maintain the air-filled chambers at a desired pressure. The Acucair® Continuous Airflow System commercially available from Hill-Rom Company, Inc. having a place of business in Batesville, Indiana is an example of such a low-air loss system that can be used as support structure 23. In each of these types of patient support structures, the air-filled chambers facilitate the equalization of the pressure used to support the patient and it is common to use such patient support structures to minimize the risk of pressure ulcers.

Conventional thermoregulating devices for chilling or warming a patient do not provide the pressure-equalization features of a patient support having air-filled chambers. Conventional thermoregulating devices often have strong polymeric exterior fabric covers that are not stretchable and do not readily conform to the contours of the patient being supported thereon. When using such conventional thermoregulating devices there may be pressure spikes at key pressure locations of the patient and these point loads will be suspended by the fabric cover of the device in a phenomenon known as "hammocking." Consequently, the benefits of a patient support structure having air-filled chambers are generally not obtainable if such a conventional thermoregulating device is placed between the patient and the patient support structure. Device 20, however, has a plurality of pressure relief sections which allow device 20 to more readily conform to the contour of the patient and thereby facilitate the equalization of the support pressure exerted by device 20 on patient 24. In other words, it facilitates the reduction of the peak pressure the patient experiences at key pressure support locations.

The illustrated support structure 23 has gas- or fluid-filled support cells 30 with a substantially cylindrical shape and vertically oriented axes 32. Support cells 30 are formed out of a polymeric material such as polyurethane and have a hollow interior volume which is filled with a gas. In the illustrated embodiment, cells 30 are filled with air. Cells 30 include a lower radial flange 31 that is welded to upper sheet 34 of plenum layer 36 using radio frequency welding to form an air tight bond. Plenum layer 36 has an interior volume that forms an air chamber or plenum. Openings (not shown) in upper sheet 34 provide fluid communication between plenum 36 and each of the support cells 30. While the cells 30 are shown in the illustrated embodiment as cylindrically-shaped, vertically oriented air bladders, the device of the present invention may be used in cooperation with other types of air bladders including more conventional horizontally oriented bladders, or with support layers filled with other materials, such as foam or three-dimensional fibers.

Support structure 23 may take the form of a powered or non-powered air surface, or a low air loss mattress. In any case, support cells 30 are generally non-permeable to air. In an exemplary air surface aspect, plenum layer 36 is also non-permeable to air and, by providing fluid communication between the inner volumes of each of the support cells 30, maintains each of the support cells 30 at substantially equivalent pressures under differing load conditions. In other words, when a large load is placed on support structure 23, the pressure within each of the support cells 30 will be raised, including those cells 30 which are not subject to the direct application of the load.

In a low air loss mattress structure, upper plenum sheet 34 permits the limited passage of air therethrough. A separate air supply is coupled to plenum 36 to provide a continuous supply of air thereto to replenish the air lost through sheet 34 and maintain plenum 36, and thus support cells 30, at a desired pressure. The flow of air toward patient 24 generated by the loss of air through upper sheet 34 beneficially removes moisture from those portions of device 20 in direct contact with patient 24.

In powered, non-powered, and low air loss embodiments, each individual support cell 30 compresses to an extent that is determined by the load placed on it. Weight loads placed on device 20 are transferred through device 20 and any intervening material layers to support cells 30. Support structure 23 includes a patient support layer 38 that is sufficiently loose and flexible so that when a patient 24 is supported on layer 38, layer 38 will generally conform to the body of patient 24 as it transfers the weight of patient 24 to support cells 30. Support cells 30 are gas-filled and the interior space of cells 30 are interconnected so that the pressure of the gas in each of the cells 30 of the mattress or a particular zone of the mattress (i.e., a head section, seat section, or foot section) remains substantially equal. Thus, as the weight of patient 24 is supported by individual support cells 30, those cells 30 which have a particularly high load initially placed thereon will be compressed toward plenum layer 36 until the load is diminished and spread to adjacent support cells 30 and the gas pressure within the cells 30 can support the load placed thereon. Those individual cells 30 that are located at the key pressure points of patient 24 will be compressed to a greater extent than the adjacent support cells 30 to thereby spread some of the weight load experienced by the most significantly compressed support cells 30 to the adjacent support cells 30. By this differential compression of the individual support cells 30, support cells 30 are designed to spread the weight load placed on apparatus 20 by patient 24 over a larger area and facilitate the reduction of the peak support pressures experienced by the patient 24.

As illustrated, patient support layer 38 covers the full length of support structure 23. A portion of layer 38 has been cutaway in Fig. 1, however, so that the underlying support cells 30 may be illustrated. Layer 38 includes a layer of ticking that forms its exposed surface and may optionally include a layer of other patient support material, such as three-dimensional material or open cell urethane foam, below the ticking. The ticking used with structure 23 is a conventional mattress ticking material. One example of suitable three-dimensional material is commercially available from Freudenberg & Co. headquartered in Weinheim an der Bergstrasse, Germany.

By providing thermoregulating device 20 with pressure relief sections which allow for the resilient, stretching of device 20, device 20 may more readily conform to the contours of patient 24 and, when placed between patient 23 and a patient support structure 23, facilitate the equalization of the internal air pressure of the bladders supporting patient 24.

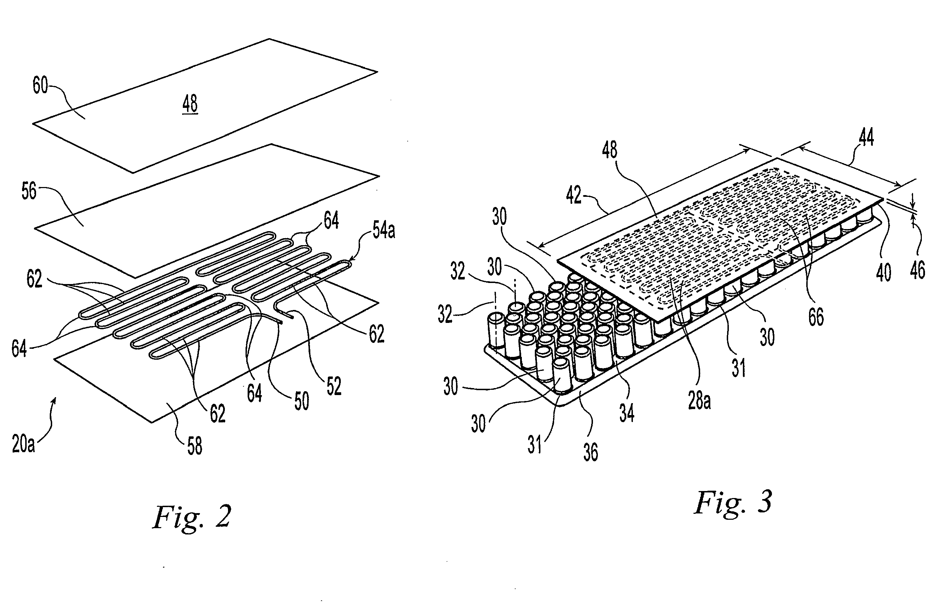

Fig. 1 illustrates the use of a thermoregulating device 20 with a bed 22, patient support structure 23 and heat exchange unit 26. Various aspects of thermoregulating device 20, i.e., devices 20a-d, are shown in greater detail in Figs. 2-19. Each of the devices 20a-20d include a flexible heat exchange layer 40 having a length 42, a width 44 and a thickness 46 wherein both the length 42 and width 44 are significantly greater than the thickness 46. The illustrated heat exchange layers 40 also all include at least one support surface 48 on which patient 24 can be supported.

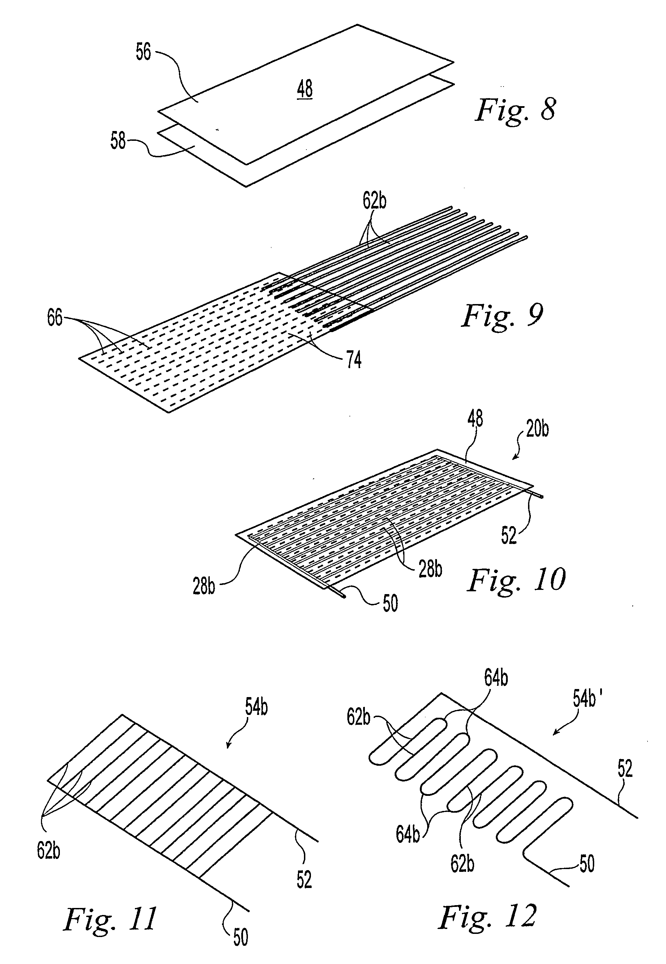

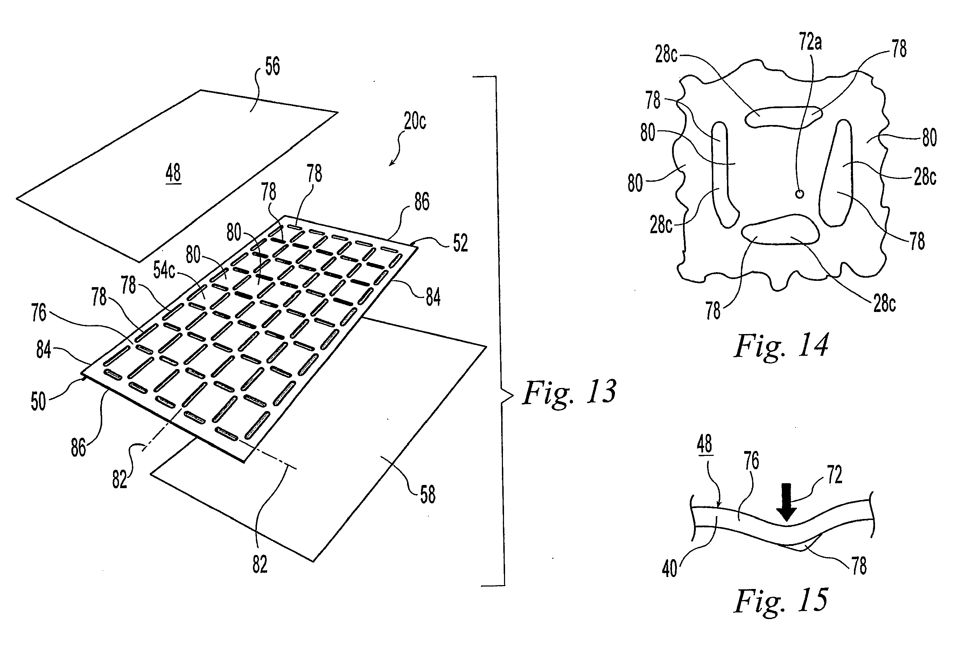

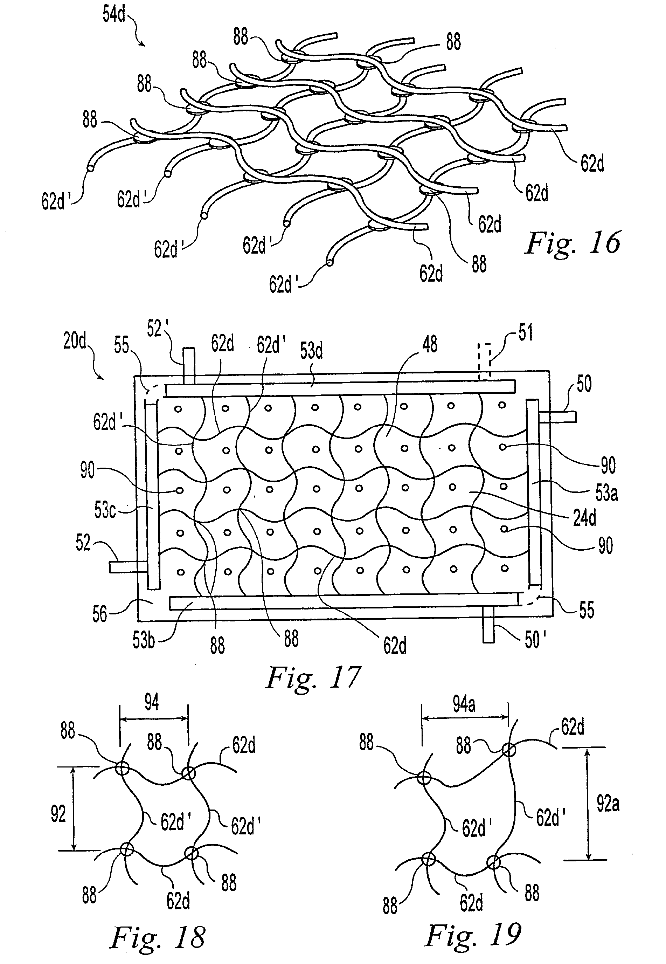

A heat exchange fluid circuit is located within layer 40 and circulates a heat exchange medium, e.g., water, to chill or warm a patient or other object placed on surface 48. Illustrated devices 20a (Figs. 2-7), 20b (Figs. 8-12), and 20d (Figs. 16-19) have heat exchange fluid circuits with elongate conduits that are formed with plastic tubes, e.g., polyvinylchloride (PVC) or polyurethane tubes, having a nominal inner diameter of about 0.25 inches (0.64 cm). Illustrated device 20c (Figs. 13-15) has a fluid circuit formed by a bladder constructed by joining two sheets of polyurethane film together with radio frequency welds.

In the illustrated aspects, water is the heat exchange medium that is circulated through the fluid circuits. Water enters the fluid circuit from heat exchange unit 26 through inlet port 50 and is discharged from the fluid circuit through outlet port 52 to return it to heat exchange unit 26. In unit 26, the water is either chilled or warmed depending upon whether it is desired to chill or warm patient 24. As the water passes through the fluid circuit within layer 40, it exchanges thermal energy with patient 24. For example, if unit 26 is used to chill the water, the water will absorb thermal energy from patient 24 thereby cooling patient 24 and warming the water.

In some situations it may be desirable to place thermoregulating devices both above and below the patient 24. In such situations, two devices 20 may be employed. For example, a device 20 consisting of only a heat exchange layer 40 may be placed on top of patient 24 and either a device 20 placed on top of a separate support structure 23 (Fig. 1) or a device 20 that includes integral support cells 30 (Fig. 3) may be placed below patient 24.

In most situations where it is desired to chill or warm a patient, it is the upper torso of the patient which is of primary concern and the illustrated heat exchange layers 40 have a length 42 and width 44 that is sufficient to cover or support the upper torso of a typical adult. Alternative aspects having either longer or shorter lengths and widths may also be employed with the present invention.

Thermoregulating device 20a is shown in Figs. 2 and 3 and includes a heat exchange fluid circuit 54a located between an upper resiliently stretchable material layer 56 and a lower resiliently stretchable material layer 58. Upper and lower layers 56, 58 are resiliently stretchable, i.e., they return to their original dimensions after being stretched. Advantageously, material layers 56, 58 may be stretched to at least about 200% of their original length or width and substantially return to their original dimensions after the force stretching the material has been removed. Material layers 56, 58 are manufactured with spandex fibers. More particularly, layers 56, 58 are a fabric material manufactured with Lycra® brand spandex fibers which are commercially available from Invista, a subsidiary of Koch Industries, Inc. having a place of business in Wichita, Kansas. Spandex fibers are well-known to those having ordinary skill in the art and are elastic, segmented polyurethane fibers that can typically be stretched to more than about 500% of their original length without breaking. Although a ticking layer 60 is positioned on top of upper material layer 56 in the illustrated aspect, ticking layer 60 can be omitted in alternative embodiments.

As seen in Figs. 2 and 3 heat exchange fluid circuit 54a is formed out of a flexible tubular member that has a generally serpentine shape. The tubular member includes a plurality of parallel linear segments 62 which are connected by arcuate portions 64. Upper and lower layers 56, 58 are stitched together to form heat exchange layer 40. Stitching 66 extends between the linear segments 62 of fluid circuit 54a to help maintain the overall configuration of fluid circuit 54a. Those portions of stretchable material layers 56, 58 located between the various segments 62 of fluid circuit 54a, however, act as pressure relief sections 28a and allow fluid circuit segments 62 to move relative to each other when a patient 24 is supported on device 20a.

As illustrated in Fig. 3, device 20a may be integrated with a patient support structure 23 having gas- or fluid-filled support cells 30. For the structure of Fig. 3, a patient support layer 38 (not illustrated in Fig. 3 but shown in part in Fig. 1) is located on top of layer 40 and extends downwardly to encircle both heat exchange layer 40 and support structure 23 so that the layer 40 and structure 23 form a single integral unit.

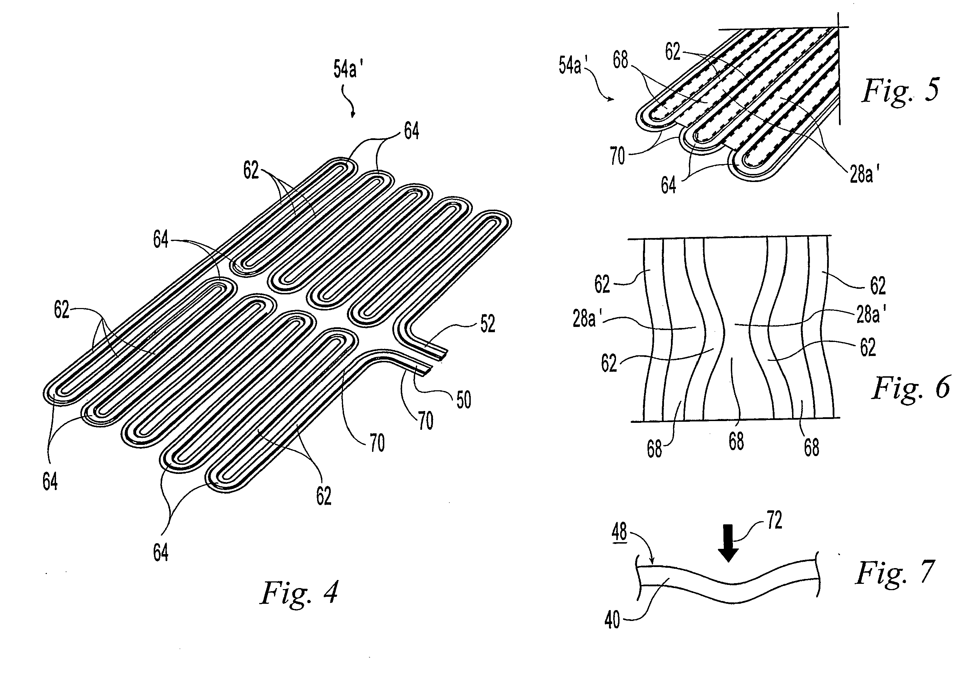

Figs. 4 and 5 illustrate an alternative aspect of the serpentine heat exchange fluid circuit of Figs. 2 and 3 wherein strips 68 of resiliently stretchable material are secured between linear segments 62 of the fluid circuit. Fluid circuit 54a' is shown in Fig. 4 and the tubular member used to form this circuit is similar to that of fluid circuit 54a but additionally includes outwardly extending flanges 70. As seen in Fig. 5, strips 68 extend between linear segments 62 and are stitched or otherwise removably (i.e., with Velcro) or non-removably coupled to flanges 70. Strips 68 are resiliently stretchable so that they allow fluid circuit segments 62 to be repositioned when subjected to force but will return segments 62 to their original positions after the force is removed. In the illustrated aspect, material strips 68 are a spandex fiber containing fabric.

Figs. 6 and 7 schematically illustrate how resiliently stretchable strips 68 function. A force 72 applied substantially transverse to surface 48 will depress layer 40 at the location where force 72 is applied. Under the application of force 72, strips 68 will elongate allowing layer 40 to deform into the shape shown in cross section in Fig. 7. This will also result in the slight repositioning of segments 62 relative to each other as schematically depicted in Fig. 6. Once force 72 is removed, strips 68 will return to their original dimensions and fluid circuit 54a' will return to its generally planar configuration depicted in Figs. 4 and 5. By facilitating this deformation of layer 40, strips 68 allow layer 40 to more readily conform to the contours of a patient 24 supported thereon.

Thermoregulating device 20b is best understood with reference to Figs. 8-12. Device 20b includes an upper and lower layer 56, 58 of resiliently stretchable material similar to device 20a (Fig. 8). The upper and lower layers 56, 58 are joined with stitching 66 to form parallel extending sleeves 74 (Fig. 9). Linear segments 62b of flexible polymeric tubing form a part of the heat exchange fluid circuit 54b and extend through sleeves 74.

Fig. 10 illustrates device 20b in its assembled condition. Those portions of stretchable material layers 56, 58 which are located between linear segments 62b form pressure relief sections 28b to allow device 20b to adapt to the contours of patient 24. As shown in Fig. 11, linear segments 62b are joined together in a parallel arrangement to form fluid circuit 54b. Fig. 12 illustrates an alternatively configured fluid circuit 54b' that connects linear fluid line segments 62b with arcuate portions 64b in series. Figs. 11 and 12 are not intended to illustrate the proportionate lengths of segments 62b.

Thermoregulating device 20c is best understood with reference to Figs. 13-15. Device 20c has a heat exchange fluid circuit 54c that is defined by a bladder 76. Bladder 76 includes a plurality of slits 78 which extend through the full thickness of bladder 76 and subdivide bladder 76 into separate fluid segments 80. Bladder 76 is formed by joining two sheets of polyurethane film together with water tight radio frequency welds along its outer edges 84, 86 and along the interior perimeter of each slit 78.

Fluid segments 80 define a number of different possible flow paths for the water circulated between inlet 50 and outlet 52. The elongate slits 78 each have an axis 82 and are arranged in a rectilinear grid pattern. In the illustrated aspect, slits 78 are arranged parallel to either the lengthwise edges 84 and or the widthwise edges 86 of bladder 76. Edges 84 and 86 of bladder 76 are positioned at right angles. Bladder 76 forms a heat exchange layer 40 by itself, however, upper and lower resiliently stretchable material layers 56, 58 may optionally be secured to bladder 76.

Slits 78 function as pressure relief sections 28c as best understood with reference to Figs. 14 and 15. When a force 72 is applied at location 72a substantially transverse to support surface 48, slits 78 are elongated as schematically depicted in Fig. 14. This differential lateral elongation of slits 78 allows layer 40 formed by bladder 76 to deform as depicted in cross section in Fig. 15 and thereby facilitate the equalization of a support pressure exerted on patient 24. When bladder 76 is returned to a generally planar position, slits 78 will be returned to their original configuration illustrated in Fig. 13.

It is noted that slits 78 do not allow the total length or width of bladder 76 to be elongated, but slits 78 do allow for the local deformation of bladder 76. When relieving support pressure spikes at key pressure locations on patient 24, local deformation of the supporting layer at the key pressure locations can facilitate the reduction of the support pressure spike even when the total length or width of bladder is not elongated or otherwise altered.

Thermoregulating device 20d is best understood with reference to Figs. 16-19. Device 20d includes a fluid circuit 54d that is formed by curvilinear fluid lines 62d, 62d' arranged in a grid pattern. The curvilinear shape of fluid lines 62d, 62d' allows each of the nodes 88 of the grid pattern to be repositioned relative to the other nodes 88 and thereby facilitate the conformance of device 20d to the contours of a patient 24 being supported thereon. Fluid circuit 54d includes a first group of generally parallel extending fluid line segments 62d and a second group of generally parallel extending fluid line segments 62d'. These first and second groups of fluid line segments extend in substantially transverse directions and intersect at grid nodes 88. Nodes 88 are formed by polymeric circular shaped tabs to which lines 62d and 62d' are each affixed. Lines 62d, 62d' may be affixed to tabs 88 by radio frequency welding, adhesives or other suitable means. As best seen in Figs. 16 and 17, each of the fluid line segments 62d and 62d' have an arcuate or curvilinear configuration that forms a generally sinusoidal path.

Two different configurations in which fluid line segments 62d, 62d' can be arranged to form fluid circuit 54d are shown in Fig. 17. The first configuration is depicted in solid lines and requires that the water pumped by heat exchanger 26 be divided into two flows. The two flows of water enter fluid circuit 54d through a first inlet 50 and a second inlet 50'. The water entering first inlet 50 is conveyed to header line 53a from which it enters fluid segments 62d. After flowing through segments 62d, where the water exchanges thermal energy with a patient 24 supported on device 20d, the water is collected in discharge header line 53c and then discharged through outlet 52 to return to heat exchanger unit 26. The second flow of water entering fluid circuit 54d passes through inlet 50' to enter header line 53b. The second flow then passes through lines 62d' where it exchanges thermal energy with patient 24 and is collected in discharge header line 53d. From discharge header line 53d, the water is discharged through outlet 52' and returns to heat exchange unit 26.

In an alternative configuration of fluid circuit 54d, header lines 53a and 53b can be joined with a corner piece 55 to form a single inlet header fed solely by inlet 50. Similarly, header lines 53c and 53d can be joined with a corner piece 55 to form a single outlet header line which discharges through a single outlet 51.

Fasteners 90 are used to secure a top and bottom resilient material layer 56, 58 together on opposite sides of fluid circuit 54d (only top layer 56 is shown in Fig. 17). Fasteners 90 are positioned within the open spaces defined by the grid pattern of fluid circuit 54d to allow for the relative movement of lines 62d, 62d'. Fasteners 90 can be formed by stitching the upper and lower stretchable material layers 56, 58 together or other suitable means. The stretchable material forming layers 56, 58 disposed about fasteners 90 and between adjacent fluid line segments form pressure relief sections 24d.

Figs. 18 and 19 schematically depict how lines 62d, 62d' and nodes 88 may be relatively repositioned as device 20d deforms when a force is applied to device 20d. Fig. 18 depicts lines 62d, 62d' and nodes 88 when device 20d is in an unstressed generally planar position. In this situation, nodes 88 are separated by a first set of distances 92,94. The curvilinear shape of flexible fluid line segments 62d and 62d' means that each fluid line segment 62d, 62d' extending between nodes 88 has a length that is greater than the linear distance between those same nodes 88 in the unstressed position depicted in Fig. 18 and thus lines 62d' and 62d define a corresponding set of lengths greater than respective distances 92, 94.

When device 20d is deformed by the application of a force, upper and lower stretchable material layers 56, 58 and the pockets formed therebetween act as pressure relief sections 24d and allow nodes 88 and fluid lines 62d, 62d' to be relatively repositioned as schematically depicted in Fig. 19. As seen in Fig. 19, upper fluid segment 62d and right hand fluid segment 62d' have been repositioned and have a more linear configuration and nodes 88 now define an altered set of distances 92a, 94a which are longer than the unstressed distances 92, 94.

## Claims

1. A thermoregulating apparatus (20, 20a-d) for exchanging thermal energy with a patient (24) supported thereon, the apparatus comprising a flexible heat exchange layer (40), the heat exchange layer defining a length (42), a width (44) and a thickness (46), each of the length and the width being substantially greater than the thickness, and a heat exchange fluid circuit (54, 54a-d, 54a') disposed within the heat exchange layer, the heat exchange fluid circuit defining at least first and second heat exchange segments, and at least one pressure relief section (28a-c, 28, 24b) disposed between the first and second heat exchange segments (62), the pressure relief segment comprising resiliently stretchable material located between the first and second heat exchange segments (62), wherein the stretchable material comprises spandex fibers, **characterized in that** the layer defines at least one support surface (48), wherein elongation of the pressure relief section relatively repositions the first and second heat exchange segments in at least one direction upon application of a force due to support of a patient on the support surface to the heat exchange layer in a direction substantially transverse to the support surface and at a location proximate the first and second heat exchange segments and the pressure relief section, and wherein the flexible heat exchange layer further comprises at least one layer of the resiliently stretchable material (56, 58) defining the support surface, the heat exchange fluid circuit disposed proximate the layer of stretchable material.

2. The apparatus of claim 1 wherein the first and second heat exchange segments each comprise an elongate conduit wherein, when the heat exchange layer is positioned in a substantially planar orientation, at least a portion of one of the conduits defining the first and second heat exchange segments has an arcuate configuration, and wherein differential elongation of the pressure relief section and the first and second heat exchange segments repositions at least a portion of the arcuate configuration into a more linear configuration.

3. The apparatus of claim 1 wherein the heat exchange fluid circuit comprises at least one elongate conduit arranged in a grid, the grid including a plurality of nodes (88), each of the nodes defining an intersection of a first segment for the conduit (62d) with a second segment of the conduit (62d') with the first segment being fixed relative to the second segment at each node, and wherein, when the heat exchange layer Is positioned in a substantially planar orientation, the nodes are separated by a first set of distances (92, 94) and the first and second segments of the conduit extending between the nodes define a second respective set of lengths, the second set of lengths being greater than the first set of distances thereby allowing the nodes to be repositionable to define a third set of distances separating the nodes that is greater than the first set of distances.

4. The apparatus of claim 3 wherein the first and second segments of the conduit have an arcuate configuration when the flexible heat exchange layer is in a generally planar configuration.

5. The apparatus of claim 4 wherein the conduit has a portion defining a generally sinusoidal configuration.

6. The apparatus of claim 1 wherein the fluid structure comprises at least one elongate conduit, the conduit having at least one substantially serpentine portion wherein, when the heat exchange layer is positioned in a substantially planar orientation, the serpentine portion defines a plurality of substantially parallel spaced apart linear segments (62) and wherein a plurality of the pressure relief sections are disposed between the linear segments of the conduit.

7. The apparatus of claim 1 wherein the first and second heat exchange segments each comprise an elongate conduit, wherein the at least one stretchable material layer defines a first sleeve (74) and a second sleeve (74), and wherein the first and second heat exchange segments are respectively disposed within the first and second sleeves.

8. The apparatus of any preceding claim wherein the flexible heat layer comprises first and second layers of the stretchable material (56, 58), the heat exchange fluid circuit disposed between the first and second layers of stretchable material.

9. The apparatus (20b) of claim 1, wherein a first sleeve portion (74) and a second sleeve portion (74) are formed within the flexible heat exchange layer, the first segment disposed within the first sleeve portion and the second segment disposed within the second sleeve portion.

10. The apparatus of claim 9 wherein the flexible heat exchange layer comprises first and second layers of stretchable material (25, 26), the first and second layers secured together to define the first and second sleeve portions and wherein at least one of the first and second layers defines the resiliently stretchable material disposed between the first segment and the second segment.

11. The apparatus of claim 9 wherein the first sleeve portion is disposed substantially parallel to the second sleeve portion.

12. The apparatus of claim 11 wherein the first and second sleeve portions are substantially linear when the flexible heat exchange layer is disposed in a planar configuration.

13. The apparatus of any preceding claim wherein the stretchable material is resiliently stretchable to at least about 200% of Its original length.

14. The apparatus of any preceding claim further comprising a support structure (23) underlying the flexible heat exchange layer, the support structure including a plurality of gas filled support cells (30).

15. The apparatus of claim 14 wherein the plurality of gas filled support cells comprise a plurality of substantially cylindrical cells, each of the cells defining an axis oriented substantially transverse to the support surface.

## Patentansprüche

1. Wärmeregelungsvorrichtung (20, 20a-d) zum Austausch von Wärmeenergie mit einem darauf befindlichen Patienten (24), wobei die Vorrichtung eine elastische Wärmeaustauschschicht (40) umfasst, wobei die Wärmeaustauschschicht eine Länge (42), eine Breite (44) und eine Dicke (46) aufweist, wobei die Länge und die Breite jeweils wesentlich größer als die Dicke ist, und einen innerhalb der Wärmeaustauschschicht angeordneten Kreislauf für ein Wärmeaustauschfluid (54, 54a-d, 54a') besitzt,
wobei der Kreislauf für ein Wärmeaustauschfluid aus mindestens ersten und zweiten Wärmeaustauschsegmenten und mindestens einem zwischen den ersten und zweiten Wärmeaustauschsegmenten (62) angeordneten Überdruckabschnitt (28a-c, 28, 24b) besteht, wobei das Überdrucksegment elastisch verstreckbares Material zwischen den ersten und zweiten Wärmeaustauschsegmenten (62) umfasst, wobei zu dem verstreckbaren Material Spandexfasern gehören,
**dadurch gekennzeichnet, dass** die Schicht mindestens eine Unterstützungsfläche (48) aufweist, wobei durch Dehnung des Überdruckabschnitts die ersten und zweiten Wärmeaustauschsegmente in mindestens einer Richtung in eine relativ neue Position gebracht werden, wenn durch Auflage eines Patienten auf der Unterstützungsfläche auf die Wärmeaustauschschicht eine Kraft in einer weitgehend quer zur Unterstützungsfläche verlaufenden Richtung und an einer Stelle in der Nähe der ersten und zweiten Wärmeaustauschsegmente und des Überdruckabschnitts wirksam wird, und wobei die elastische Wärmeaustauschschicht des Weiteren mindestens eine die Unterstützungsfläche bildende Schicht aus dem elastisch verstreckbaren Material (56, 58) aufweist, wobei der Kreislauf für das Wärmeaustauschfluid in der Nähe der Schicht aus verstreckbarem Material angeordnet ist.

2. Vorrichtung nach Anspruch 1, wobei die ersten und zweiten Wärmeaustauschsegmente jeweils eine dehnbare Leitung umfassen, wobei im Fall einer Positionierung der Wärmeaustauschschicht mit einem weitgehend planen Verlauf mindestens ein Teil einer der Leitungen, welche die ersten und zweiten Wärmeaustauschsegmente bilden, eine bogenförmige Konfiguration hat, und wobei durch eine unterschiedliche Dehnung des Überdruckabschnitts und der ersten und zweiten Wärmeaustauschsegmente mindestens ein Teil der bogenförmigen Konfiguration in eine mehr lineare Konfiguration neu positioniert wird.

3. Vorrichtung nach Anspruch 1, wobei der Kreislauf für das Wärmeaustauschfluid mindestens eine in einem Gitter angeordnete dehnbare Leitung umfasst, wobei zum Gitter eine Vielzahl von Knotenpunkten (88) gehören, die jeweils eine Schnittstelle eines ersten Segments für die Leitung (62d) mit einem zweiten Segment der Leitung (62d') bilden, wobei das erste Segment an jedem Knotenpunkt relativ zum zweiten Segment befestigt ist, und wobei die Knotenpunkte, wenn die Wärmeaustauschschicht in einer im wesentlichen planen Richtung verläuft, durch eine erste Reihe von Abständen (92, 94) voneinander getrennt sind und die zwischen den Knotenpunkten verlaufenden ersten und zweiten Segmente der Leitung eine entsprechende zweite Reihe von Abständen bilden, wobei die zweite Reihe von Längen größer als die erste Reihe von Abständen ist, so dass die Knotenpunkte in eine andere Position gebracht werden können, um eine dritte Reihe von die Knotenpunkte voneinander trennenden Abständen zu erhalten, die größer als die erste Reihe von Abständen sind.

4. Vorrichtung nach Anspruch 3, wobei die ersten und zweiten Segmente der Leitung eine bogenförmige Konfiguration haben, wenn die elastische Wärmeaustauschschicht eine im allgemeinen plane Konfiguration aufweist.

5. Vorrichtung nach Anspruch 4, wobei die Leitung einen Abschnitt umfasst, der eine allgemein sinusförmige Konfiguration hat.

6. Vorrichtung nach Anspruch 1, wobei die Fluidanordnung mindestens eine dehnbare Leitung umfasst, wobei die Leitung mindestens einen weitgehend serpentinenförmig verlaufenden Abschnitt aufweist, wobei der serpentinenförmig verlaufende Abschnitt eine Vielzahl von weitgehend parallel voneinander abgesetzten linearen Segmenten (62) bildet, wenn die Wärmeaustauschschicht sich in einer weitgehend planen Ausrichtung befindet, und wobei eine Vielzahl von Überdruckabschnitten zwischen den linearen Segmenten der Leitung angeordnet sind.

7. Vorrichtung nach Anspruch 1, wobei die ersten und zweiten Wärmeaustauschsegmente jeweils eine dehnbare Leitung umfassen, wobei die mindestens eine Schicht aus verstreckbarem Material eine erste Muffe (74) und eine zweite Muffe (74) bildet und wobei die ersten und zweiten Wärmeaustauschsegmente jeweils innerhalb der ersten und zweiten Muffen angeordnet sind.

8. Vorrichtung nach irgendeinem der vorhergehenden Ansprüche, wobei die elastische Wärmeaustauschschicht erste und zweite Schichten des verstreckbaren Materials (56, 58) umfasst, wobei der Kreislauf für das Wärmeaustauschfluid zwischen den ersten und zweiten Schichten aus verstreckbarem Material angeordnet ist.

9. Vorrichtung (20b) nach Anspruch 1, wobei ein erstes Muffenteil (74) und ein zweites Muffenteil (74) innerhalb der elastischen Wärmeaustauschschicht ausgebildet sind, wobei das erste Segment innerhalb des ersten Muffenteils angeordnet ist und das zweite Segment sich innerhalb des zweiten Muffenteils befindet.

10. Vorrichtung nach Anspruch 9, wobei die elastische Wärmeaustauschschicht erste und zweite Schichten aus verstreckbarem Material (25, 26) umfasst, wobei die ersten und zweiten Schichten miteinander verbunden sind, um erste und zweite Muffenteile zu bilden, und wobei mindestens eine der ersten und zweiten Schichten das zwischen dem ersten Segment und dem zweiten Segment angeordnete elastisch verstreckbare Material ist.

11. Vorrichtung nach Anspruch 9, wobei das erste Muffenteil weitgehend parallel zum zweiten Muffenteil angeordnet ist.

12. Vorrichtung nach Anspruch 11, wobei die ersten und zweiten Muffenteile weitgehend linear sind, wenn die elastische Wärmeaustauschschicht eine plane Konfiguration hat.

13. Vorrichtung nach irgendeinem der vorhergehenden Ansprüche, wobei das verstreckbare Material auf mindestens etwa 200 % seiner ursprünglichen Länge elastisch gestreckt werden kann.

14. Vorrichtung nach irgendeinem der vorhergehenden Ansprüche mit des Weiteren einer Stützkonstruktion (23) unterhalb der elastischen Wärmeaustauschschicht, wobei die Stützkonstruktion eine Vielzahl von gasgefüllten Stützzellen (30) umfasst.

15. Vorrichtung nach Anspruch 14, wobei die Vielzahl gasgefüllter Stützzellen eine Vielzahl von weitgehend zylindrischen Zellen umfasst und jede der Zellen eine Achse bildet, die im Wesentlichen quer zur Unterstützungsfläche verläuft.

## Revendications

1. Dispositif de thermorégulation (20, 20a-d) pour échanger l'énergie thermique avec un patient (24) supporté sur ce dernier, l'appareil comprend une couche d'échange thermique souple (40), la couche d'échange thermique définissant une longueur (42), une largeur (44) et une épaisseur (46), chacune parmi la longueur et la largeur étant sensiblement supérieure à l'épaisseur, et un circuit de fluide d'échange thermique (54, 54a-d, 54a') disposé à l'intérieur de la couche d'échange thermique, le circuit de fluide d'échange thermique définissant au moins des premier et second segments d'échange thermique, et au moins une section de limitation de pression (28a-c, 28, 24b) disposée entre les premier et second segments d'échange thermique (62), le segment de limitation de pression comprenant un matériau élastiquement extensible positionné entre les premier et second segments d'échange thermique (62), dans lequel le matériau extensible comprend des fibres de Spandex, **caractérisé en ce que** la couche définit au moins une surface de support (48), dans lequel l'allongement de la section de limitation de pression repositionne relativement les premier et second segments d'échange thermique dans au moins une direction, suite à l'application d'une force, due au support d'un patient sur la surface de support, sur la couche d'échange thermique dans une direction sensiblement transversale par rapport à la surface de support et à un emplacement à proximité des premier et second segments d'échange thermique et de la section de limitation de pression, et dans lequel la couche d'échange thermique souple comprend en outre au moins une couche du matériau élastiquement extensible (56, 58) définissant la surface de support, le circuit de fluide d'échange thermique étant disposé à proximité de la couche de matériau extensible.

2. Appareil selon la revendication 1, dans lequel les premier et second segments d'échange thermique comprennent chacun un conduit allongé dans lequel, lorsque la couche d'échange thermique est positionnée dans une orientation sensiblement plane, au moins une partie de l'un des conduits définissant les premier et second segments d'échange thermique a une configuration arquée, et dans lequel l'allongement différentiel de la section de limitation de pression et des premier et second segments d'échange thermique repositionne au moins une partie de la configuration arquée dans une configuration plus linéaire.

3. Appareil selon la revendication 1, dans lequel le circuit de fluide d'échange thermique comprend au moins un conduit allongé agencé dans une grille, la grille comprenant une pluralité de noeuds (88), chacun des noeuds définissant une intersection d'un premier segment pour le conduit (62d) avec un second segment du conduit (62d'), avec le premier segment qui est fixe par rapport au second segment au niveau de chaque noeud, et dans lequel, lorsque la couche d'échange thermique est positionnée dans une orientation sensiblement plane, les noeuds sont séparés par un premier ensemble de distances (92, 94) et les premier et second segments du conduit s'étendant entre les noeuds définissent un deuxième ensemble respectif de longueurs, le second ensemble de longueurs étant supérieur au premier ensemble de distances, permettant ainsi aux noeuds de pouvoir être repositionnés afin de définir un troisième ensemble de distances séparant les noeuds qui est supérieur au premier ensemble de distances.

4. Appareil selon la revendication 3, dans lequel les premier et second segments du conduit ont une configuration arquée lorsque la couche d'échange thermique souple est dans une configuration généralement plane.

5. Appareil selon la revendication 4, dans lequel le conduit a une partie définissant une configuration généralement sinusoïdale.

6. Appareil selon la revendication 1, dans lequel la structure de fluide comprend au moins un conduit allongé, le conduit ayant au moins une partie sensiblement en serpentin, dans lequel, lorsque la couche d'échange thermique est positionnée dans une orientation sensiblement plane, la partie de serpentin définit une pluralité de segments linéaires (62) espacés sensiblement parallèles et dans lequel une pluralité de sections de limitation de pression sont disposées entre les segments linéaires du conduit.

7. Appareil selon la revendication 1, dans lequel les premier et second segments d'échange thermique comprennent chacun un conduit allongé, dans lequel la au moins une couche de matériau extensible définit un premier manchon (74) et un second manchon (74), et dans lequel les premier et second segments d'échange thermique sont respectivement disposés à l'intérieur des premier et second manchons.

8. Appareil selon l'une quelconque des revendications précédentes, dans lequel la couche d'échange thermique souple comprend des première et seconde couches de matériau extensible (56, 58), le circuit de fluide d'échange thermique étant disposé entre les première et seconde couches de matériau extensible.

9. Appareil (20b) selon la revendication 1, dans lequel une première partie de manchon (74) et une seconde partie de manchon (74) sont formées à l'intérieur de la couche d'échange thermique souple, le premier segment étant disposé à l'intérieur de la première partie de manchon et le second segment étant disposé à l'intérieur de la seconde partie de manchon.

10. Appareil selon la revendication 9, dans lequel la couche d'échange thermique souple comprend des première et seconde couches de matériau extensible (25, 26), les première et seconde couches étant fixées ensemble afin de définir les première et seconde parties de manchon et dans lequel au moins l'une des première et seconde couches définit le matériau élastiquement extensible disposé entre le premier segment et le second segment.

11. Appareil selon la revendication 9, dans lequel la première partie de manchon est disposée de manière sensiblement parallèle par rapport à la seconde partie de manchon.

12. Appareil selon la revendication 11, dans lequel les première et seconde parties de manchon sont sensiblement linéaires lorsque la couche d'échange thermique souple est disposée dans une configuration plane.

13. Appareil selon l'une quelconque des revendications précédentes, dans lequel le matériau extensible est élastiquement extensible jusqu'à au moins environ 200% de sa longueur d'origine.

14. Appareil selon l'une quelconque des revendications précédentes, comprenant en outre une structure de support (23) sous-jacente par rapport à la couche d'échange thermique souple, la structure de support comprenant une pluralité de cellules de support (30) remplies de gaz.

15. Appareil selon la revendication 14, dans lequel la pluralité de cellules de support remplies de gaz comprennent une pluralité de cellules sensiblement cylindriques, chacune des cellules définissant un axe orienté de manière sensiblement transversale par rapport à la surface de support.
